# EUROPEAN PATENT APPLICATION

(11) **EP 4 130 747 A1**
(43) Date of publication of application: **08.02.2023**
(21) Application number: 21780387.3
(22) Date of filing: 29.03.2021
(51) Int. Cl.: G01N 33/68, G01N 33/574, C12Q 1/6886

(54) **BIOMARKER FOR CANCER IMMUNOTHERAPY AND USE THEREOF**

(30) Priority: 30.03.2020 KR 20200038620; 26.03.2021 KR 20210039303
(71) Applicant: Rophelbio Co., Ltd.a, Seoul 04778 (KR)
(72) Inventor: CHOI, Seonghee, Yecheon-gun, Gyeongsangbuk-do 36815 (KR); KIM, Jong-Han, Seoul 04778 (KR); CHU, Hun Su, Seoul 04195 (KR); JEONG, Won-Jin, Chuncheon-si, Gangwon-do 24214 (KR)
(74) Representative: Dehns
(86) International application number: PCT/KR2021/003834
(87) International publication number: WO 2021/201526

(57) **Abstract**

DRG2 of the present invention is a predictor of a therapeutic response or prognosis of a cancer patient with respect to an agent for cancer immunotherapy in PD-L1 positive cancer and is a biomarker that can be used in companion diagnosis for determination of the administration of an agent for cancer immunotherapy in PD-L1 positive cancer. By determining whether to administer an agent for cancer immunotherapy through the present invention, the agent for cancer immunotherapy can be selectively administered to a patient who will show a therapeutic response, and thus is expected to treat PD-L1 positive cancer more effectively.

## Description

### [Technical Field]

The present invention relates to a biomarker for cancer immunotherapy and use thereof.

### [Background Art]

Tumor cells act on host immunity in several ways to evade immune defenses in the tumor microenvironment. This phenomenon is generally referred to as "cancer immune escape". One of the most important components in this system is an immunosuppressive co-signal (immune checkpoint) mediated by the programmed cell death protein 1 (PD-1) receptor and its ligand, programmed death-ligand (PD-L1).

PD-1 receptors and PD-L1 ligands play essential roles in immunoregulation. PD-1, which is expressed on activated T cells, is activated by PD-L1 and PD-L2, which are expressed by stromal cells, tumor cells, or both, and through this, T-cell death and localized immune suppression are initiated to potentially provide an immune-tolerant environment for tumor development and growth. Conversely, inhibition of this interaction can enhance local T cell responses and mediate antitumor activity (Iwai Y, et al. Proc Natl Acad Sci USA 2002; 99: 12293-97).

Therefore, cancer immunotherapy, particularly, an immune checkpoint inhibitor, has recently been developed as a therapy for killing cancer cells by activating the immune system of the human body, and is used for cancer treatment. As the most widely used immune checkpoint inhibitors, there are inhibitors using monoclonal antibodies against PD-1 or PD-L1. These inhibitors eliminate cancer cells by suppressing the binding of PD-1 on the surface of T cells and PD-L1 on the surface of cancer cells to activate immune cells such as T cells, and it has been reported that these inhibitors dramatically increase the survival rate of cancer patients, and some patients do not have cancer recurrence for several years.

Inhibitors using monoclonal antibodies against PD-1 and PD-L1 are only effective against cancer cells expressing PD-L1. Therefore, these inhibitors are applied only to cancer expressing PD-L1 by analyzing the expression of PD-L1 in cancer tissues before PD-1 and PD-L1 inhibitors are used. However, it has been reported that 50% or more of cancer patients with cancer expressing PD-L1 do not respond to PD-1 and PD-L1 inhibitors, but the reason is still unknown. Therefore, in order to effectively treat cancer using an agent for cancer immunotherapy, which targets PD-1 and PD-L1, the reason that cancer cells expressing PD-L1 do not respond to PD-1 and PD-L1 inhibitors should be clarified, and there is an emerging need for selecting patients who show a high therapeutic effect on PD-1 and PD-L1 inhibitors based on this.

### [Disclosure]

### [Technical Problem]

The present invention has been devised to solve the aforementioned technical problems in the related art, and it is an object of the present invention to provide a composition or kit for predicting a therapeutic response for an agent for cancer immunotherapy, or a method for providing information on a therapeutic response or prognosis prediction for an agent for cancer immunotherapy using the same, and the like.

More specifically, the present inventors confirmed that when the expression level of a developmentally-regulated GTP-binding protein 2 (DRG2) gene or DRG2 protein is decreased, T cells capable of killing cancer cells are activated despite the expression of PD-L1 in cancer cells. Therefore, the present inventors confirmed that the expression levels of DRG2 are associated with both the expression of PD-L1 and the activation of apoptotic T cells in cancer cells and that it is possible to predict a therapeutic response for an agent for cancer immunotherapy against PD-L1 positive cancer by measuring the expression level of DRG2, thereby completing the present invention.

Therefore, an object of the present invention is to provide a composition or kit for predicting a therapeutic response or prognosis for an agent for cancer immunotherapy.

Another object of the present invention is to provide a composition or kit for companion diagnosis for predicting a therapeutic response or prognosis for an agent for cancer immunotherapy in PD-L1 positive cancer.

Still another object of the present invention is to provide a method for providing information for predicting a therapeutic response or prognosis for an agent for cancer immunotherapy.

Yet another object of the present invention is to provide a method for providing information for predicting a therapeutic response or prognosis for an agent for cancer immunotherapy in PD-L1 positive cancer.

However, the technical problems which the present invention intends to solve are not limited to the technical problems which have been mentioned above, and other technical problems which have not been mentioned will clearly be understood by a person with ordinary skill in the art to which the present invention pertains from the following description.

### [Technical Solution]

The present invention provides a composition for predicting a therapeutic response or prognosis for an agent for cancer immunotherapy, including a preparation that measures the level of a developmentally-regulated GTP-binding protein 2 (DRG2) gene or DRG2 protein.

Further, the present invention provides a kit for predicting a therapeutic response or prognosis for an agent for cancer immunotherapy, including a composition for predicting a therapeutic response or prognosis for an agent for cancer immunotherapy.

Furthermore, the present invention provides a composition for companion diagnosis for predicting a therapeutic response or prognosis for an agent for cancer immunotherapy in PD-L1 positive cancer, including a preparation that measures the level of the DRG2 gene or DRG2 protein.

In addition, the present invention provides a kit for companion diagnosis for predicting a therapeutic response or prognosis for an agent for cancer immunotherapy in PD-L1 positive cancer, including the composition for companion diagnosis for predicting a therapeutic response or prognosis for an agent for cancer immunotherapy in PD-L1 positive cancer.

In an exemplary embodiment of the present invention, the preparation which measures the level of the DRG2 gene may be preferably a primer or probe that specifically binds to the DRG2 gene, but is not limited thereto as long as the preparation is a preparation capable of measuring the expression level of the DRG2 gene by amplifying the entire DRG2 gene sequence or a part thereof.

In another exemplary embodiment of the present invention, the preparation which measures the level of the DRG2 protein may be preferably an antibody or aptamer that specifically binds to the DRG2 protein, and the antibody may be the entire antibody or a part thereof, but the preparation is not limited thereto as long as the preparation is a preparation capable of binding to the DRG2 protein to measure the amount of the protein.

In still another exemplary embodiment of the present invention, the agent for cancer immunotherapy may be a drug that specifically binds to programmed cell death protein 1 (PD-1) or programmed death-ligand 1 (PD-L1), but is not limited thereto.

In yet another exemplary embodiment of the present invention, the drug that specifically binds to PD-1 may be an anti-PD-1 antibody, and may be preferably pembrolizumab, nivolumab, cemiplimab, and the like, but is not limited as long as the drug is a drug known to specifically bind to PD-1 to serve as an agent for cancer immunotherapy.

In yet another exemplary embodiment of the present invention, the drug that specifically binds to PD-L1 may be an anti-PD-L1 antibody, and may be preferably atezolizumab, avelumab, durvalumab, and the like, but is not limited as long as the drug is a drug known to specifically bind to PD-L1 to serve as an agent for cancer immunotherapy.

In yet another exemplary embodiment of the present invention, the composition may be for predicting a therapeutic response or prognosis for an agent for cancer immunotherapy against PD-L1 positive cancer. The PD-L1-positive cancer may be, for example, cancer that is determined to be positive for PD-L1 expression through an immunohistochemistry test, but is not limited thereto as long as the PD-L1 positive cancer is PD-L1 positive cancer determined by a method for determining PD-L1 positive cancer, which is generally used.

In yet another exemplary embodiment of the present invention, the composition may predict cancer with decreased expression levels of the DRG2 gene or DRG2 protein compared to a control as cancer with a low therapeutic response to an agent for cancer immunotherapy or cancer with a poor prognosis, but is not limited thereto. The control group may have an expression level of the DRG2 gene or DRG2 protein in a biological sample isolated from a normal person, that is, a person without cancer, or an expression level of the DRG2 gene or DRG2 protein measured in normal tissue around cancer tissue, not in cancer tissue.

In yet another exemplary embodiment of the present invention, the composition may predict PD-L1 positive cancer with decreased expression levels of the DRG2 gene or DRG2 protein compared to the control as cancer with a low therapeutic response to an agent for cancer immunotherapy or cancer with a poor prognosis, but is not limited thereto.

In yet another exemplary embodiment of the present invention, the decreased expression of the DRG2 gene or DRG2 protein may indirectly show a decrease in the level of PD-L1 expressed on the surface of cancer cells, which may show a decreased amount of binding between cancer cell PD-L1 and T cell PD-1, or may show an increased level of PD-L1 in cancer cells, and an increase in the number of CD8 T cells or activity thereof, but is not limited thereto.

In yet another exemplary embodiment of the present invention, the level of the DRG2 gene or DRG2 protein may be preferably measured in a biological sample isolated from a cancer patient, the biological sample is cancer cells, cancer tissue, blood, plasma, serum, bone marrow, saliva, urine, stool, and the like, preferably cancer cells or cancer tissue, but is not limited thereto as long as the biological sample is a sample including the DRG2 gene or DRG2 protein.

Further, the present invention provides a method for providing information for predicting a therapeutic response or prognosis for an agent for cancer immunotherapy, the method including: measuring the level of a DRG2 gene or DRG2 protein in a biological sample isolated from a cancer patient.

Furthermore, the present invention provides a method for providing information for companion diagnosis for an agent for cancer immunotherapy in PD-L1 positive cancer, the method including: measuring the level of a DRG2 gene or DRG2 protein in a biological sample isolated from a PD-L1 positive cancer patient.

In an exemplary embodiment of the present invention, when the level of the DRG2 gene or DRG2 protein is decreased compared to the control, it may be predicted that the therapeutic response to the agent for cancer immunotherapy is low or the prognosis is poor, but the prediction is not limited thereto.

Further, the present invention provides a method for treating cancer, the method including: a) measuring the level of a DRG2 gene or DRG2 protein in a biological sample isolated from a cancer patient; b) selecting a patient in which a level of the DRG2 gene or DRG2 protein is decreased compared to a control; and c) administering an agent for cancer immunotherapy to the selected patient.

Further, the present invention provides a use of a composition including a preparation which measures the level of a developmentally-regulated GTP-binding protein 2 (DRG2) gene or DRG2 protein for predicting a therapeutic response or prognosis for an agent for cancer immunotherapy.

Further, the present invention provides a use of a composition including a preparation which measures the level of a developmentally-regulated GTP-binding protein 2 (DRG2) gene or DRG2 protein for selecting a patient for administering an agent for cancer immunotherapy.

### [Advantageous Effects]

DRG2 of the present invention is a predictor of a therapeutic response or prognosis of a cancer patient with respect to an agent for cancer immunotherapy in PD-L1 positive cancer and is a biomarker that can be used in companion diagnosis for determination of the administration of an agent for cancer immunotherapy in PD-L1 positive cancer. Specifically, after the amount of DRG2 present inside and/or on the surface of tissue cells in PD-L1 positive cancer is measured, the amount of DRG2 measured can be analyzed to determine whether or not to administer an agent for cancer immunotherapy. By determining whether to administer an agent for cancer immunotherapy through the selection method of the present invention, the agent for cancer immunotherapy can be selectively administered to patients who will show a high therapeutic effect, and thus is expected to treat PD-L1 positive cancer more effectively.

### [Description of Drawings]

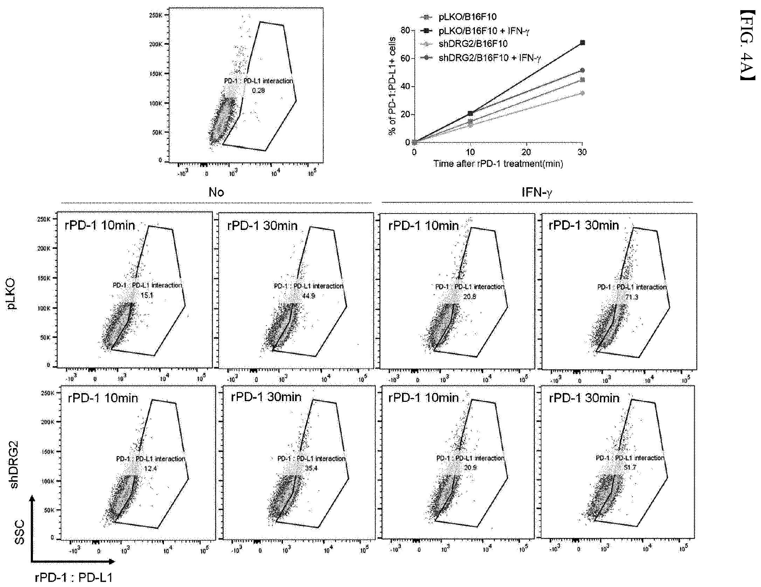

FIG 1 illustrates the correlation between high PD-L1 expression and low DRG2 expression in a clinical database.
Specifically, FIG 1A illustrates high PD-L1 (CD274) gene expression in TCGA tumors and overall survival based on GTEx data using GEPIA (survival curve).
FIG 1B illustrates low PD-L1 (CD274) gene expression in TCGA tumors and overall survival based on GTEx data using GEPIA (survival curve).
FIGS. 2A to 2E show that DRG2 deficiency enhances the activity of CD8 T cells in a melanoma tumor model.
Specifically, FIG 2A illustrates the results of DRG2 knock-down confirmed by qRT-PCR (left) and western blot (right).
FIG 2B illustrates the results of observing the growth of melanoma for 18 days after subcutaneously injecting a control and DRG2 KD melanoma (5 × 10⁵ cells) into C57BL/6 mice.
FIG 2C illustrates flow cytometry results of CD8⁺IFN-γ⁺ T cells, CD3⁻NK1.1⁺ NK cells, CD11C⁺F4/80⁺ M1 MΦ and CD206⁺F4/80⁺ M2 MΦ in tumor infiltrating lymphocytes (TILs).
FIG 2D illustrates the results of measuring the levels of T cell immune checkpoints such as inhibitory ligands PD-L1, PD-L2 and Gal-9, activating ligands 4-1BBL, OX40L and CD70, and inhibitory and activating ligands CD80 and CD86 in a primary control and DRG2 KD primary melanoma by qRT-PCR.
FIG 2E illustrates the western blot results (left) of antibody-treated primary melanoma lysates and the flow cytometry results (right) for PD-L1 levels.
FIGS. 3A to 3F show that DRG2 deficiency increases the expression of IFN-γ-induced PD-L1 in melanoma through enhancement of the IFN-γ pathway.
Specifically, FIGS. 3A and 3B illustrate qRT-PCR results showing changes in PD-L1 expression by IFN-γ treatment (5 ng/ml) for 24 hours in the control and DRG2 KD B16F10 cells, and flow cytometry results for PD-L1 levels by histograms.
FIG 3C illustrates the western blot results of IFN-γ-STAT1 signaling in the control and DRG2 KD B16F10 cells treated with IFN-γ for 24 hours in a dose-dependent manner.
FIS. 3D illustrates the results of qRT-PCR analysis of PD-L1 against the IRF1 transcription factor.
FIG 3E illustrates the soluble IL-2 levels of the control and activated primary CD4 T cells co-cultured with DRG2 KD B16F10 cells, which are pre-treated or not treated with IFN-γ for 48 hours in flat-bottom 96-well plates (left) and 24 transwell plates (right).
FIG 3F illustrates levels of IL-2 mRNA (left) and soluble IL-2 (right) of activated primary CD4T cells cultured with the supernatant of the control and DRG2 KD B16F10 cells.
FIGS. 4A to 4C show that PD-L1 has reduced binding affinity with recombinant PD-1 in DRG2-deficient melanoma.
Specifically, FIG 4A illustrates flow cytometry results for interactions between recombinant PD-1 and PD-L1 in melanoma. The control and DRG2 KD melanoma cells were treated or not treated with IFN-γ (0.5 ng/ml) for 24 hours and then treated with recombinant PD-1 (1 µg/ml) for 10 minutes 30 minutes prior to flow cytometry.

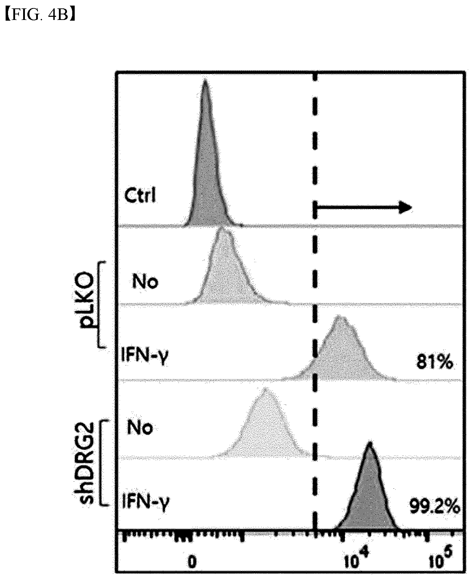

FIG 4B illustrates the flow cytometry histogram of the control and DRG2 KD melanoma cells treated and untreated with IFN-γ (0.5 ng/ml) for 24 hours for PD-L1. The interaction affinity between PD-1 and PD-L1 was calculated as % of PD-1⁺ to PD-L1⁺ cells.

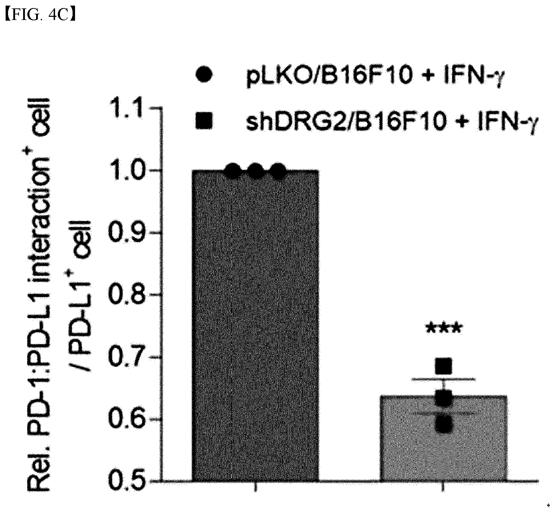

FIG 4C illustrates the ratio of cells interacting with PD-1 per PD-L1⁺ cells in the control and DRG2 KD cells.

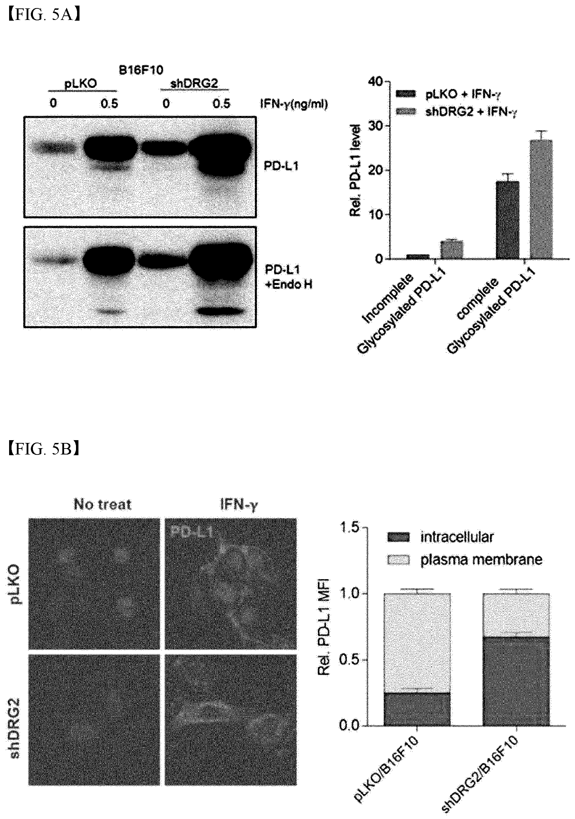

FIGS. 5A to 5C show that DRG2 deficiency increases intracellular PD-L1.
Specifically, FIG 5A illustrates the western blot results (left) for PD-L1 deglycosylation according to the Endo H treatment in the control and DRG2 KD B16F10 cell lysates treated or untreated with IFN-γ and the corresponding graph (right).
FIG 5B illustrates a confocal microscope image (left) showing the protein expression of PD-L1 after IFN-γ treatment or non-treatment in the control and DRG2 KD melanoma cells and the corresponding graph (right). Red fluorescence indicates PD-L1, and blue fluorescence indicates nuclei.

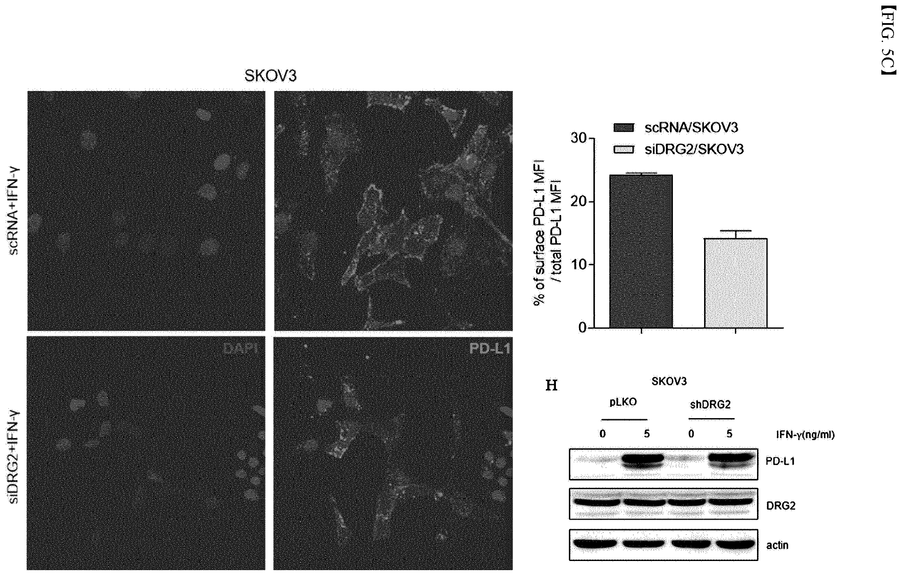

FIG 5C illustrates a confocal microscope image (left) showing the protein expression of PD-L1 after IFN-γ treatment in the control and DRG2 KD human ovarian cancer cells (SKOV3), the corresponding graph (right upper row) showing the ratio of PD-L1 expressed on the cell surface and the western blot results (right lower row) confirming the effects of DRG2 KD and IFN-γ. Red indicates PD-L1, and blue indicates nuclei.

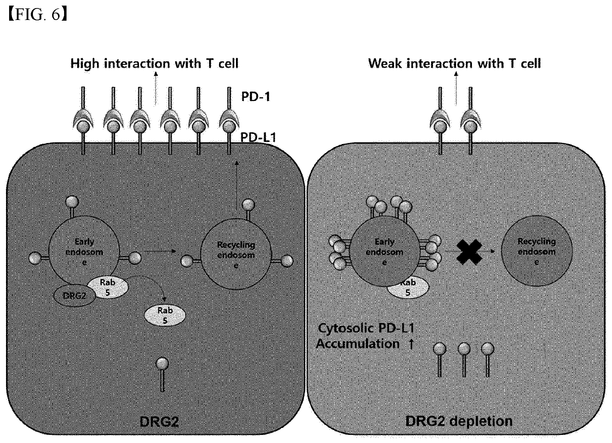

FIG 6 is a view schematically illustrating the changes in the expression level of PD-L1 on the surface of cancer cells according to changes in the expression level of DRG2, and the corresponding changes in the interaction between PD-L1 on the surface of cancer cells and PD-1 of T cells.

### [Modes of the Invention]

The present inventors focused on the fact that when an agent for cancer immunotherapy is administered to PD-L1-positive cancer patients, the agent for cancer immunotherapy exhibits therapeutic effects only in 20 to 30% of cancer patients, and have made intensive studies on a method for selecting a patient for administering an agent for cancer immunotherapy. As a result, they confirmed that PD-L1 is not normally located on the cell surface in the case of patients with decreased expression of a DRG2 gene or DRG2 protein, resulting in a lower therapeutic effect of the agent for cancer immunotherapy, thereby completing the present invention. More specifically, in general, it was confirmed that when the expression of PD-L1 was increased, the survival rate was decreased due to a decrease in the activation of immune cells such as T cells, but when the expression of the DRG2 gene or DRG2 protein was decreased, the expression of PD-L1 was increased, but the growth of cancer was suppressed. In this regard, as a result of a detailed study, it was confirmed that when the expression of the DRG2 gene or DRG2 protein was decreased, PD-L1 whose expression was increased was not located normally on the cell surface, and thus, the degree of binding to PD-1 was decreased. That is, although an agent for cancer immunotherapy against PD-1 or PD-L1 exhibits the efficacy of the agent for cancer immunotherapy by suppressing the binding of PD-1 and PD-L1, it could be confirmed that the agent for cancer immunotherapy cannot act on cancer in which the expression level of the DRG2 gene or DRG2 protein is decreased. That is, it could be confirmed that the existing method of measuring only the expression level of PD-L1 and administering an agent for cancer immunotherapy cannot accurately select a patient capable of showing a therapeutic effect with respect to an agent for cancer immunotherapy. Therefore, the present invention remarkably enhance a therapeutic efficiency of an agent for cancer immunotherapy by providing a method of selecting a cancer patient in which the expression of the DRG2 gene or DRG2 protein is increased or a cancer patient who is a PD-L1 positive cancer patient and simultaneously has increased expression of the DRG2 gene or DRG2 protein as a patient to be administered an agent for cancer immunotherapy, and thus is expected to effectively reduce the pain and treatment costs of the cancer patient.

In the present specification, developmentally-regulated GTP-binding protein 2 (DRG2) is a protein involved in various intracellular regulations, and is known to form a large superfamily, this superfamily has three important subfamilies: trimeric G protein (for example, transducin), monomeric G protein (for example, ras), and GTPase involved in protein synthesis (for example, elongation factor Tu), recently, a new group of G proteins with different base sequences and functions from these subfamilies was discovered. And a developmentally regulated GTP-binding protein (DRG), which is one of them, has all five regions from G1 to G5 required for binding to GTP, and is similar in size to trimeric G protein. However, DRG has completely different amino acid sequence characteristics from not only trimeric G protein but also existing G protein subfamilies, and thus, is classified as a new subfamily.

Further, DRG includes DRG1 and DRG2, and is present in almost all types of cells from bacteria to humans. For example, it was confirmed that DRG is present in Halobacterium cutirubrum, Thermoplasma acidophilum, Methanococcus jannaschii, Caenorhabditis elegans, Schizosaccharomyces pombe, Drosophila melanogaster, Xenopus laevis, Arabidopsis thaliana, Saccharomyces cerevisiae, and the like. However, it is not yet known that DRG2 is associated with the expression of PD-L1 in cancer cells and the activation of apoptotic T cells.

In the present specification, the level (or expression level) of the gene or protein of DRG2 is used in a sense to include both the mRNA expression level of the DRG2 gene and the expression level of the DRG2 protein expressed therefrom. In the present specification, the level (expression level) of the gene is a process of confirming the presence or absence of mRNA of the DRG2 gene and/or the degree of expression thereof in a biological sample in order to predict a therapeutic effect of an agent for cancer immunotherapy, and can be confirmed by measuring the amount of mRNA. An analysis method for this includes a reverse transcription polymerase chain reaction (RT-PCR), competitive RT-PCR, real-time RT-PCR, an RNase protection assay (RPA), northern blotting, RNA-sequencing (RNA-seq), nanostrings, DNA microarray chips, and the like, but is not limited thereto as long as the method is a method capable of measuring the expression level of mRNA. In addition, in the present specification, the level (expression level) of the protein is a process of confirming the presence or absence and expression level of a protein encoded by the DRG2 gene in a biological sample in order to predict the therapeutic effect of an agent for cancer immunotherapy, and can be found by confirming the amount of the protein using an antibody or aptamer that specifically binds to the protein or by measuring the activity of the protein. An analysis method for this includes western blotting, enzyme linked immunosorbent assay (ELISA), radioimmunoassay, radioimmunodiffusion, Ouchterlony immunodiffusion, Rocket immunoelectrophoresis, immunohistochemical staining, immunoprecipitation assay, complete fixation assay, fluorescence activated cell sorting (FACS), protein chips, ligand binding assay, matrix assisted laser desorption/ionization time of flight mass spectrometry (MALDI-TOF) analysis, surface enhanced laser desorption/ionization time of flight mass spectrometry (SELDI-TOF) analysis, 2-dimensional electrophoresis analysis, liquid chromatography-mass spectrometry (LC-MS), liquid chromatography-mass spectrometry/mass spectrometry (LCMS/MS), and the like, but is not limited thereto as long as the method is a method capable of measuring the expression level of a protein.

As used herein, "companion diagnosis" refers to one of the diagnostic tests for identifying the feasibility of applying a specific therapeutic drug to a specific patient, and in the present invention, the expression level of DRG2 in cancer patients can be measured as a companion diagnostic marker in order to confirm the possibility of applying an agent for cancer immunotherapy (for example, an anti-PD-1 antibody) to a subject with PD-L1 positive cancer.

As used herein, "method of providing information for predicting a therapeutic response or prognosis for an agent for cancer immunotherapy" relates to a method for predicting the therapeutic effect of an agent for cancer immunotherapy and predicting a prognosis through the prediction, and refers to a method for obtaining information on the possibility that the therapeutic effect of the agent for cancer immunotherapy on patients with decreased expression of the DRG2 gene or DRG2 protein may be low.

In the present specification, the preparation which measures the level of the DRG2 gene may be a primer or probe that specifically binds to the DRG2 gene, but is not limited thereto. The primer or probe has a sequence complementary to the biomarker (DRG2) nucleotide sequence of the present invention. As used herein, the term "complementary" refers to having sufficient complementarity that can selectively hybridize to the above-described nucleotide sequence under any specific hybridization or annealing conditions. Therefore, as the term "complementary" has a meaning different from the term "perfectly complementary", the primer or probe of the present invention can have one or more mismatched base sequences while being capable of selectively hybridizing to the above-described nucleotide sequence.

As used herein, the term "primer" refers to a single-stranded oligonucleotide which may act as an initiation point of synthesis with a template sequence under suitable conditions (that is, four types of different nucleoside triphosphates and polymerases) in a suitable buffer solution at a suitable temperature. A suitable length of the primer may vary according to various factors, for example, the temperature and the use of the primer, but is typically 15 to 30 nucleotides. A short primer molecule generally requires a lower temperature to form a sufficiently stable hybrid complex with a template. Primers may be easily designed by those skilled in the art by referencing the above-described nucleotide sequence, and may be produced using, for example, a primer design program (for example: PRIMER 3 program).

As used herein, the term "probe" refers to a linear oligomer of natural or modified monomers or linkages, may include deoxyribonucleotides and ribonucleotides, may specifically hybridize to a target nucleotide sequence, and is naturally occurring or artificially synthesized.

In the present specification, the preparation which measures the level of the DRG2 protein may be an antibody or aptamer that specifically binds to the DRG2 protein, but is not limited thereto.

The antibody means recognizing a part or all of the DRG2 protein as an antigenic site to specifically and directly bind to the antigenic site, and includes all of a polyclonal antibody, a monoclonal antibody, or fragments thereof. Such antibodies may be produced by publicly-known methods known to those skilled in the art. That is, the monoclonal antibody may be produced using a fusion method, a recombinant DNA method, or a phage antibody library technique widely known in the art.

The aptamer is a single-stranded DNA or RNA molecule, and may be obtained by separating an oligomer that binds to a specific chemical molecule or biological molecule with high affinity and selectivity by an evolutionary method using an oligonucleotide library called systematic evolution of ligands by exponential enrichment (SELEX). The aptamer can specifically bind to a target to regulate the activity of the target, and can, for example, block the ability of the target to function through binding.

As used herein, "agent for cancer immunotherapy" refers to a material that completely or partially inhibits, interferes with or regulates one or more immune checkpoint proteins, also called immune checkpoint inhibitors. The immune checkpoint protein regulates the activation or function of T cells. A number of immune checkpoint proteins, for example, PD-1, PD-L1, CTLA-4 and the like, are known. These proteins are involved in the co-stimulatory or inhibitory interactions of T cell responses. An agent for cancer immunotherapy may include an antibody or may be derived from an antibody.

In the present specification, the agent for cancer immunotherapy may be a drug that specifically binds to programmed cell death protein 1 (PD-1), but is not limited thereto. In a specific embodiment, the drug that specifically binds to PD-1 may be an anti-PD-1 antibody, and examples of the anti-PD-1 antibody include pembrolizumab, nivolumab, cemiplimab, and the like.

In the present specification, the agent for cancer immunotherapy may be a drug that specifically binds to programmed cell death-ligand 1 (PD-L1), but is not limited thereto. In a specific embodiment, the drug that specifically binds to PD-L1 may be an anti-PD-L1 antibody, and examples of the anti-PD-L1 antibody include atezolizumab, avelumab, durvalumab, and the like.

The "antibody" is a material that specifically binds to immune checkpoint proteins such as PD-1, PD-L1 and CTLA4 to exhibit immune checkpoint inhibitory activity. The scope of the antibody includes not only an intact antibody, but also an antigen-binding site of the antibody molecule.

As used herein, cancer refers to a malignant solid tumor that is predicted to grow indefinitely along with various hematological cancers that originate from stem cells in hypoxic bone marrow that can expand locally by infiltration and systematically through metastasis. Although not particularly limited thereto, specific examples of cancer include adrenal cancer, bone cancer, brain cancer, breast cancer, bronchial cancer, colon and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of cancer include adenocarcinoma, adenoma, basal cell carcinoma, cervical dysplasia and epithelial cancer, Ewing's sarcoma, squamous cell carcinoma, axillary cell carcinoma, malignant brain tumors, blastoma, intestinal ganglion neuroma, hyperplastic corneal nerve cancer, islet cell carcinoma, Kaposi cancer, leiomyoma, leukemia, lymphoma, malignant carcinoid, malignant melanoma, malignant hypercalcemia, Marpanoid habitus cancer, myeloid cancer, metastatic skin cancer, mucosal neuroma, myelodysplastic syndrome, myeloma, filamentous sarcoma, neuroblastoma, osteosarcoma, osteogenic and other sarcomas, ovarian cancer, pheochromocytoma, polycythemia vera, primary brain tumors, small cell lung cancer, ulcerative and papillary squamous cell carcinoma, seminoma, soft tissue sarcoma, retinoblastoma, rhabdomyoblastoma, renal cell tumors or renal cell carcinoma, reticulum cell sarcoma, and Wilms' tumors. Specific examples of cancer also include astrocytoma, gastrointestinal stromal tumor (GIST), glioma or glioblastoma, renal cell carcinoma (RCC), hepatocellular carcinoma (HCC), and pancreatic neuroendocrine cancer. Preferably, the cancer may be selected from the group consisting of lung cancer, gastric cancer, glioma, liver cancer, melanoma, renal cancer, urothelial carcinoma, head and neck cancer, Merkel-cell carcinoma, prostate cancer, hematologic malignancy, breast cancer, colorectal cancer, colon cancer, rectal cancer, pancreatic cancer, brain cancer, ovarian cancer, bladder cancer, bronchial cancer, skin cancer, cervical cancer, endometrial cancer, esophageal cancer, thyroid cancer, bone cancer and a combination thereof, but is not limited thereto.

In the present invention, the composition of the present invention may be for predicting a therapeutic response or prognosis for an agent for cancer immunotherapy in PD-L1 positive cancer, but is not limited thereto. The PD-L1-positive cancer may be a cancer that is determined to be positive for PD-L1 expression through immunohistochemistry assay, and a method for determining PD-L1-positive or -negative cancer through immunohistochemistry assay and a kit therefor (for example, PD-L1 IHC 22C3 pharmDx, which is a companion diagnostic kit of KEYTRUDA^{™}) are known.

The PD-L1 companion diagnostic kit (for example, PD-L1 IHC 22C3 pharmDx) stains both PD-L1 in the cell membrane and cytoplasm during staining to determine the expression degree of PD-L1, and is used in a manner of using an agent for cancer immunotherapy (for example, Keytruda) as a therapeutic agent in the case of PD-L1 positive cancer (tumor proportion score ≥ 50%) based on this. That is, the determination of whether to administer an agent for cancer immunotherapy is based on the proportion of cancer cells expressing PD-L1 in cancer tissue. Cancer PD-L1 has a mechanism of suppressing CD8 T cells, thereby further promoting the growth of cancer. Conversely, the agent for cancer immunotherapy is a method of treating a cancer patient by suppressing PD-L1, thereby increasing the activity of T cells. In order to perform such treatment, PD-L1 needs to be well expressed in cancer tissue. When the agent for cancer immunotherapy is administered to a cancer patient whose PD-L1 expression is poor, there is almost no therapeutic effect.

In the present specification, "PD-L1 positive" may mean at least about 1% PD-L1 expression. The expression of PD-L1 may be measured by any method known in the related art. For example, the expression of PD-L1 may be measured by immunohistochemistry (IHC). As a result of being measured by IHC, the PD-L1 positive cancer (tumor) may be a cancer in which at least about 1%, at least about 2%, at least about 5%, at least about 10%, or at least about 20% of tumor cells express PD-L1.

In the present invention, the kit of the present invention refers to a diagnostic device capable of predicting the therapeutic effect of an agent for cancer immunotherapy by measuring the level of the DRG2 gene or DRG2 protein in a sample, and is not limited as long as the kit is in a form capable of confirming the amount of DRG2 gene or DRG2 protein in a biological sample isolated from a cancer patient. The kit may be in the form of a gene amplification kit, a microarray chip, and the like, but is not limited thereto. The term "amplification" refers to a reaction that amplifies nucleic acid molecules. Various amplification reactions have been reported in the art, for example the polymerase chain reaction (PCR) is disclosed in US Pat. Nos. 4683195, 4683202, and 4800159. In the microarray, a probe may be included, and the probe is used as a hybridizable array element and immobilized on a substrate. A preferred substrate is a suitable rigid or semi-rigid support, and examples thereof may include a membrane, a filter, a chip, a slide, a wafer, a fiber, a magnetic or non-magnetic bead, a gel, tubing, a plate, a polymer, a microparticle, and a capillary tube. The aforementioned hybridizable array element is arranged and immobilized on the gas. Such immobilization may be carried out by a chemical bonding method or a covalent bonding method such as UV. A sample applied to the microarray of the present invention may be labeled, and is hybridized with array elements on the microarray. Hybridization conditions may vary. The detection and analysis of the degree of hybridization may be variously performed depending on the labeling materials. In addition, the kit of the present invention may additionally include housekeeping gene, beta-actin, and the like as a control.

As used herein, the term "reference value or control" refers to a reference value that distinguishes the overexpression/underexpression of a gene (mRNA) or protein. The reference value may be, for example, the average mRNA/protein expression level of a normal person, or the average mRNA/protein expression level of normal tissue surrounding cancer tissue, but is not limited thereto. In addition, the reference value or control may be determined by the distribution of the average mRNA/protein expression level of a specific patient group, but is not limited thereto.

Hereinafter, preferred examples for helping with understanding of the present invention will be suggested. However, the following examples are provided only so that the present invention may be more easily understood, and the content of the present invention is not limited by the following examples.

### [Examples]

### Experimental materials and experimental methods

### Cell Culture

Mouse melanoma B16F1 and B16F10 cell lines and a human ovarian cancer SKOV3 cell line were purchased from Korean Cell Line Bank (KCLB-Seoul, Korea). Cells were cultured in a DMEM medium supplemented with 10% bovine fetal serum (WELGENE, Korea) at a temperature of 37°C under a humidified atmosphere of 5% CO₂. The effect of hypoxia on gene expression in the B16F10 cell line was tested by incubating the cells in a multi-gas incubator (Galaxy R, New Brunswick Scientific) that maintains a gas mixture consisting of 93% N₂, 5% CO₂ and 2% O₂.

### 1-1. Experimental animal

6-week-old female C57BL/6 mice were purchased from Orient Bio (Busan, Korea). The mice were bred in a laboratory animal facility of the University of Ulsan under specific pathogen-free conditions, and were used according to the guidelines of the Animal Care and Use Committee of University of Ulsan.

### TCGA (Cancer Genome Atlas) data analysis

Overall survival (OS) analysis was performed based on PD-L1 (CD274) gene expression in TCGA tumors compared to TCGA normal and GTEx data using Gene Expression Profiling Interactive Analysis (GEPIA). In regard to hypothesis testing, GEPIA considered a log-rank test. CD274 high and low expression cohorts were classified based on a CD274 expression threshold of 50% (median). Therefore, samples with CD274 expression levels higher or lower than 50% were classified into high expression cohorts (cutoff-high) and low expression cohorts (cutoff-low), respectively. Data including DRG2 expression profiles and clinical information of several patients were obtained using Gene Expression Profiling Interactive Analysis (GEPIA). Sample levels (Fragments Per Kilobase of transcript per Million (FPKM) normalization) were obtained.

### Plasmid, siRNA, transfection and reporter assay

A plasmid construct pLKO-DRG2-shRNA including shRNA (DRG2-shRNA, TRC0000047195, 5'-CCGGGCTCATCCTACATGAATACAACTCGAGTTGTATTCATGTAGGATGAG CTTTTTG-3' (SEQ ID NO: 25)) for mouse DRG2 and a non-target shRNA control vector (MISSION pLKO.1-puro non-mammalian shRNA control plasmid DNA, SHC002) were purchased from Sigma. The siRNA (siDRG2, 5'-CAUUGAAUACAAAGGUGCCAACA-3' (SEQ ID NO: 26)) for mouse/humans and a control siRNA (scRNA) were purchased from GenePharma.

To produce DRG2-deficient cells, B16F10 cells were transfected with pLKO-DRG2-shRNA, and B16F10/shDRG2 were selected using puromycin (Sigma P9620). Control cells B16F10/PLKO were produced using non-target shRNA in pLKO.1-puro. Cells were transfected using TurboFect (Thermo Scientific). To monitor the efficiency of transfection, the GFP expression vector pEGFP-N1/C1 (Clontech) was co-transfected with the plasmid construct. After the transfection efficiency (> 80%) was confirmed, the cells were used for further studies.

### Real-time and semiquantitative RT-PCR

Total RNA was extracted from cells using a TRIzol reagent (Invitrogen). After the concentration of RNA was measured using the NanoDrop^{™} 2000 system (Thermo Fisher Scientific, Waltham, MA, USA), cDNA was synthesized using 2 µg of total RNA and M-MLV reverse transcriptase (Promega) and used as a template in real-time and semiquantitative PCR using the gene-specific primers shown in Table 1. Real-time qRT-PCR was performed with an ABI 7500 Fast Real-Time PCR system (Applied Biosystems) using SYBR Green PCR Master Mix (QIAGEN). Semiquantitative RT-PCR was performed using Taq polymerase (Solgent, Daejeon, Korea).

**[Table 1]**

| Gene name | Forward Primer | Reverse Primer |
|---|---|---|
| mouse DRG2 | TGGAACCATCCAAATCCGCC (SEQ ID NO: 1) | CAAGAAGGTGGACTTACCCACA (SEQ ID NO: 2) |
| mouse PD-L1 | AGCCTGGAACAACGGACATT (SEQ ID NO: 3) | CTGCTAAGCCAGGAACCCTC (SEQ ID NO: 4) |
| mouse PD-L2 | TTATTCACCGTGACAGCCCC (SEQ ID NO: 5) | AGTGCATTCTCTGCGGTCAA (SEQ ID NO: 6) |
| mouse Gal9 | GTGCAGTTCTCTCAGCCAGT (SEQ ID NO: 7) | GTGGGCAGGACGAAAGTTCT (SEQ ID NO: 8) |
| mouse CD80 | TCAATACGACTCGCAACCACA (SEQ ID NO: 9) | GAGGGTCTTCTGGGGGTTTT (SEQ ID NO: 10) |
| mouse 4-1BBL | ACCTGGGTACCCGAGAGAAT (SEQ ID NO: 11) | GTAGCTTGGCGAACACAGGA (SEQ ID NO: 12) |
| mouse OX40L | AGAAGACGCTAAGGCTGGTG (SEQ ID NO: 13) | GCCGGAGAGGAAGAGAGTTG (SEQ ID NO: 14) |
| mouse CD70 | CCGCACACAGCTGAGTTACA (SEQ ID NO: 15) | CTCTGGTCCGTGTGTGAAGG (SEQ ID NO: 16) |
| mouse CD86 | AAAGAGGAGCAAGCAGACGC (SEQ ID NO: 17) | CATGGTGCATCTGGGGTCCAT (SEQ ID NO: 18) |
| mouse IRF1 | GAAAGTCCAAGTCCAGCCGA (SEQ ID NO: 19) | GCTGTGGTCATCAGGTAGGG (SEQ ID NO: 20) |
| mouse IL-2 | GAAACTCCCCAGGATGCTCA (SEQ ID NO: 21) | CGCAGAGGTCCAAGTTCATCT (SEQ ID NO: 22) |
| mouse GAPDH | CCACTCACGGCAAATTCAAC (SEQ ID NO: 23) | CTCCACGACATACTCAGCAC (SEQ ID NO: 24) |

### Western blotting

For deglycosylation assay, proteins in cell extracts or concentrated supernatants were separated by SDS-PAGE, and treated with anti-DRG2 (Proteintech), mouse anti-PD-L1 (Abcam), anti-phospho STAT1 (Cell Signaling) and anti-β-actin (Sigma) dilution solutions, respectively. Immunoreactivity bands were detected using a Pierce ECL Western blotting substrate (Thermo Scientific).

### PD-1 and PD-L1 interaction assay

To measure PD-1 and PD-L1 protein interactions, suspension cells were immobilized with 4% paraformaldehyde at room temperature for 15 minutes and incubated with a recombinant human PD-1 Fc protein (R&D Systems) for 1 hour. An anti-human Alexa Fluor 488 dye conjugate (Life Technologies) was used as a secondary antibody. Nuclei were stained with DAPI (blue; Life Technologies). Then, the fluorescence intensity of Alexa Fluor 488 dye was measured using a FACS flow cytometry analyzer (Becton Dickinson, Inc.).

### Immunocytochemistry

For immunocytochemical analysis, cells were immobilized with 4% paraformaldehyde at room temperature for 15 minutes, permeabilized with 5% TritonX-100 for 5 minutes, and then stained using a primary antibody. As a secondary antibody, an anti-mouse Alexa Fluor 488 or 594 dye conjugate and/or an anti-rabbit Alexa Fluor 488 or 594 dye conjugate (Life Technologies) were/was used. Nuclei were stained with 4',6-diamidino-2-phenylindole (DAPI; blue; Life Technologies). After mounting, cells were observed using an Olympus 1000/1200 laser-scanning confocal system.

### Co-culture and IL-2 measurement

To analyze the effect of tumor cells on T-cell inactivation, tumor cells were co-cultured with mouse spleen CD4 T cells activated with mouse T-Activator CD3/CD28 (Life Technologies). The tumor cells were incubated at a ratio of 5 : 1 (Jurkat : tumor cell) for 48 hours. The level of IL-2 secreted into the medium was measured using a mouse IL-2 ELISA kit (Thermo Scientific).

### Statistical significance

All experiments were repeated at least three times, and the results are shown as mean ± standard deviation. Statistical significance was confirmed by a Student's t-test, and when P < 0.05, it was determined to be statistically significant. Ns indicates non-significant, * indicates P < 0.05, ** indicates P < 0.01, *** indicates P < 0.001, and **** indicates P < 0.0001.

### Experimental results

### Example 1. Confirmation of association between PD-L1 and survival rate

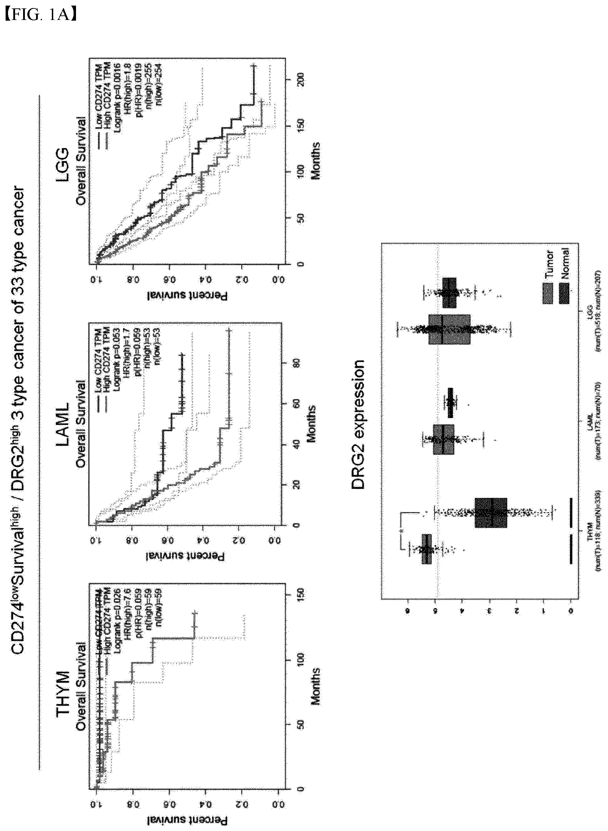

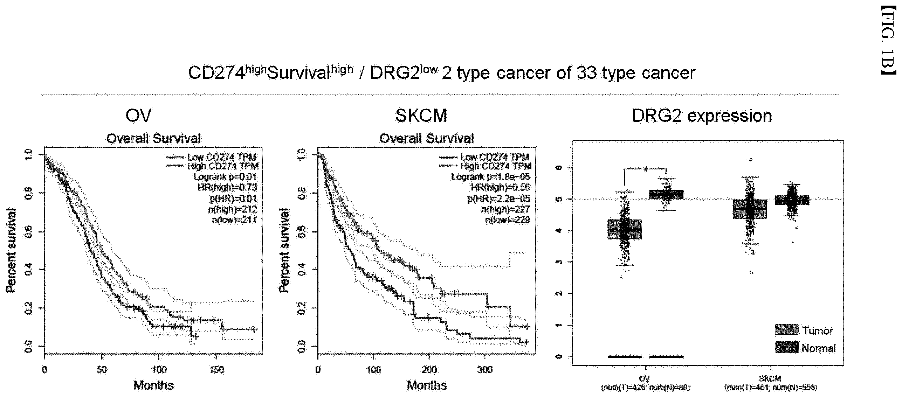

As is known, it was confirmed using clinical data whether a low survival rate was shown when the level of PD-L1 was high. The results are illustrated in FIGS. 1A and 1B.

As illustrated in FIG 1A, it was confirmed that cancer patients with high PD-L1 levels exhibited low survival rates. As a result of confirming the expression level of DRG2 in such patients, it was confirmed that the expression of DRG2 was increased in cancer cells compared to normal surrounding tissues.

In contrast, as illustrated in FIG 1B, as a result of confirming the expression level of DRG2 in patients with high levels of PD-L1, but high survival rates, the expression of DRG2 was reduced in cancer cells compared to normal surrounding tissues.

Through this, it could be confirmed that high levels of PD-L1 do not necessarily show a low survival rate, as previously known, and that the survival rate varies depending on the expression degree of DRG2.

### Example 2. Confirmation of cancer growth and immune cell distribution according to DRG2 expression level

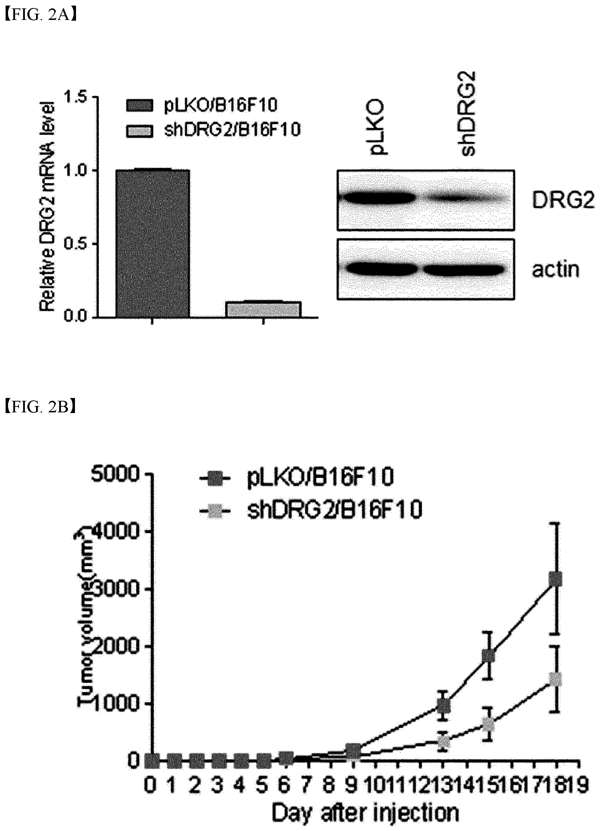

To confirm the effect of DRG2 expression level on cancer, cancer was injected into mice, and cancer growth and immune cell distribution were confirmed. The results are illustrated in FIGS. 2A to 2E.

FIG 2A illustrates the results of qRT-PCR (left) and western blot (right) experiments showing that levels of DRG2 were reduced in DRG2 knock-down melanoma. As illustrated in FIG 2A, it was confirmed that cells transfected with shDRG2 had decreased DRG2 expression.

FIG 2B illustrates the results of measuring cancer growth over time after subcutaneous injection of cells (pLKOB 16F 10 or shDRG2B 16F 10) into the flank of mice. As illustrated in FIG 2B, it was confirmed that when cancer cells (shDRG2B 16F 10) with reduced levels of DRG2 were injected into the mice, cancer growth was suppressed compared to the control (PLKO/B16F10).

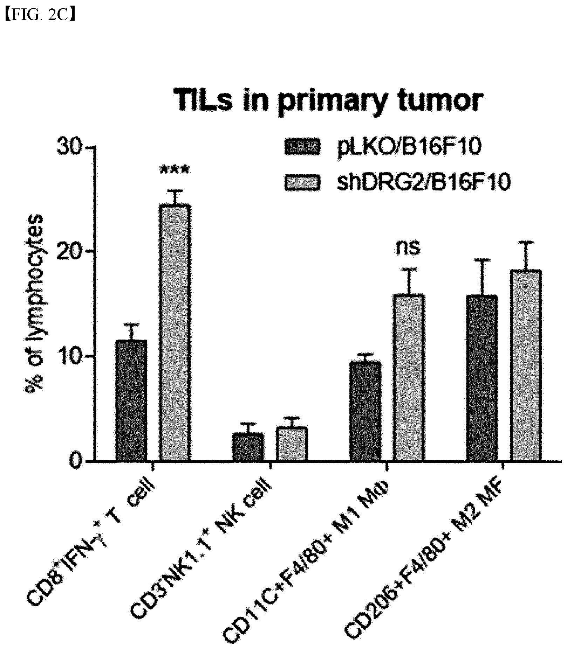

Flow cytometry was performed to confirm whether the differences in cancer growth described above were due to differences in the distribution of immune cells around the cancer (FIG. 2C). Various immune cells are present around cancer, and the main cell that serves to directly kill cancer is the apoptotic T cell (CD8T cell). Therefore, the distribution of CD8⁺IFN-gamma⁺ T cells, an activated form of CD8 T cells, in cancer tissues with reduced levels of DRG2 was confirmed. The results are illustrated in FIG 2C. As illustrated in FIG 2C, it was confirmed that CD8⁺IFN-gamma⁺ T cells were significantly increased in shDRG2B16F10 cancer tissues, but other immune cells (cells that indirectly affect CD8 T cells) had no difference from the control. Through this, it was confirmed that when the level of DRG2 in cancer cells was decreased, cancer cells could not regulate the activity of CD8 T cells, and such activity regulation was not achieved by a mechanism by other immune cells, but directly achieved through interaction with CD8 T cells.

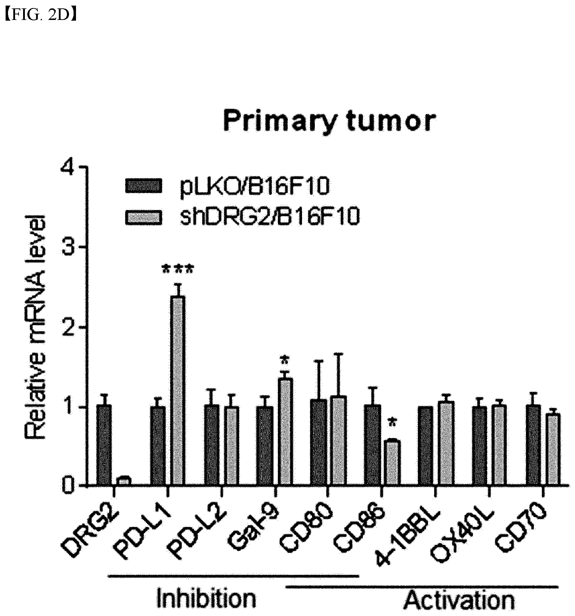

The amount of surface proteins of cancer tissue, which play an important role in regulating T cells, was confirmed at the mRNA level by qRT-PCR. The results are illustrated in FIG 2D. As illustrated in FIG 2D, as a result of confirming both the surface proteins that activate and suppress T cells in cancer tissues with decreased levels of DRG2, it was confirmed that the expression level of the PD-L1 gene, which suppresses the activity of T cells, was increased.

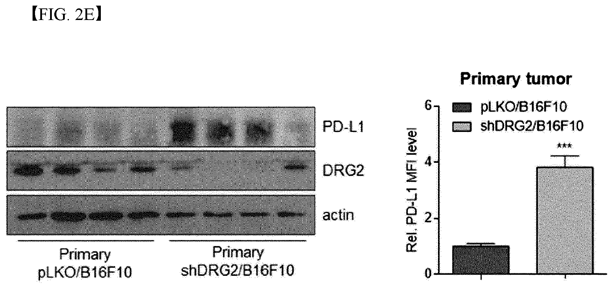

Western blot and flow cytometry were performed to confirm whether the same effect was exhibited at the protein level. The results are illustrated in FIG 2E. As illustrated in FIG 2E, similar to the mRNA results, it was confirmed that PD-L1 protein levels were increased in cancer tissues in which the level of DRG2 was decreased.

Through the above results, it is generally known that an increase in expression of PD-L1 in cancer tissues suppresses the activation of T cells to promote the growth and metastasis of cancer, and it could be confirmed that the level of PD-L1 was increased in cancer tissues in which DRG2 levels were decreased, but T cells were activated to suppress the growth of cancer.

### Example 3. Confirmation of effect of DRG2 expression level on expression of PD-L1

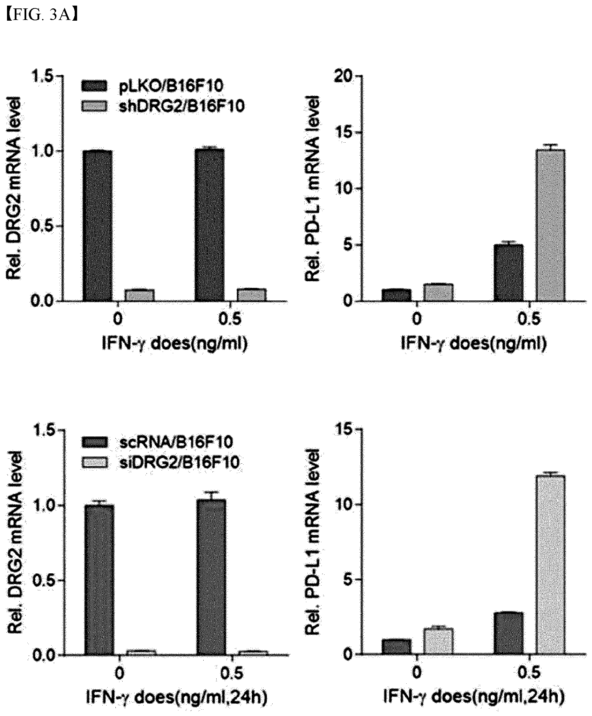

*In vitro* experiments were performed to confirm the direct effect of reduced levels of DRG2 on PD-L1. The results are illustrated in FIGS. 3A to 3F. To mimic the interaction between T cells and cancer tissue, the medium was co-treated with the cytokine IFN-gamma, which increases the expression of PD-L1, and the experiment was performed.

The upper graph in FIG 3A shows the reduction in the level of DRG2 using shDRG2, and the lower graph shows the reduction in the level of DRG2 using siDRG2. After the level of DRG2 was artificially reduced, the level of the PD-L1 gene induced by IFN-gamma was confirmed by qRT-PCR. As illustrated in FIG 3A, it was confirmed that when the level of DRG2 was reduced, the expression of the PD-L1 gene was increased.

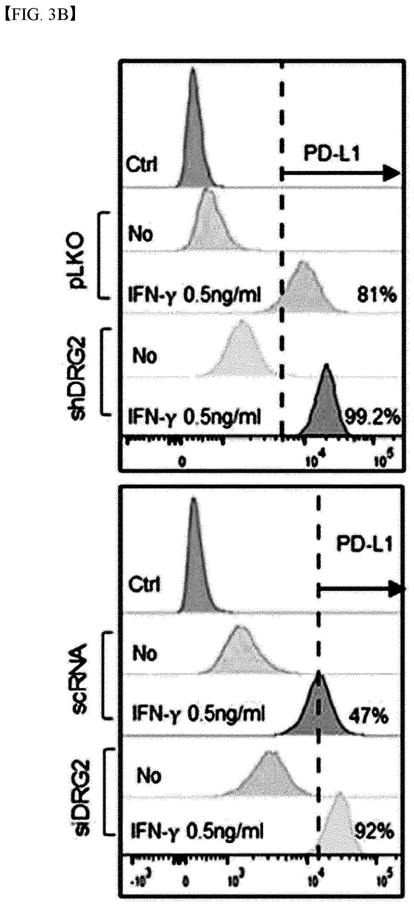

Further, the level of the PD-L1 protein was confirmed through flow cytometry. The results are illustrated in FIG 3B. As illustrated in FIG 3B, it was confirmed that the level of DRG2 was reduced, the amount of PD-L1 protein was increased.

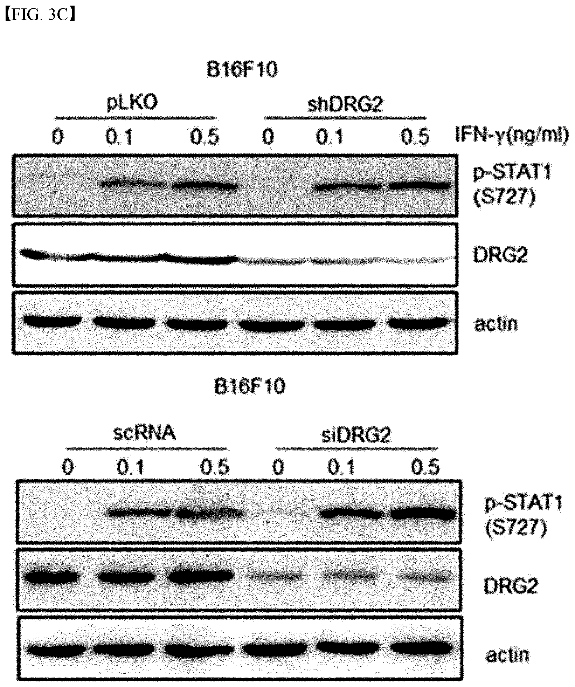

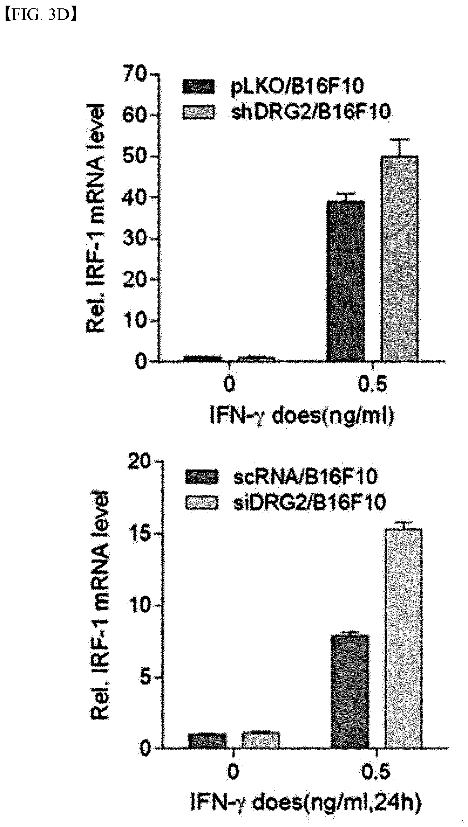

FIGS. 3C and 3D illustrate the results of confirming STAT1 phosphorylation which is a main activation pathway of IFN-gamma and the expression degree of IRF1 by western blot and qRT-PCR, respectively in order to confirm the mechanism by which the level of PD-L1 is increased when the level of DRG2 is decreased. As illustrated in FIGS. 3C and 3D, it was confirmed that a decrease in the level of DRG2 remarkably increased STAT1 phosphorylation and the expression degree of IRF1 by IFN-gamma. In addition, through this, it was confirmed that the expression of PD-L1 could be increased.

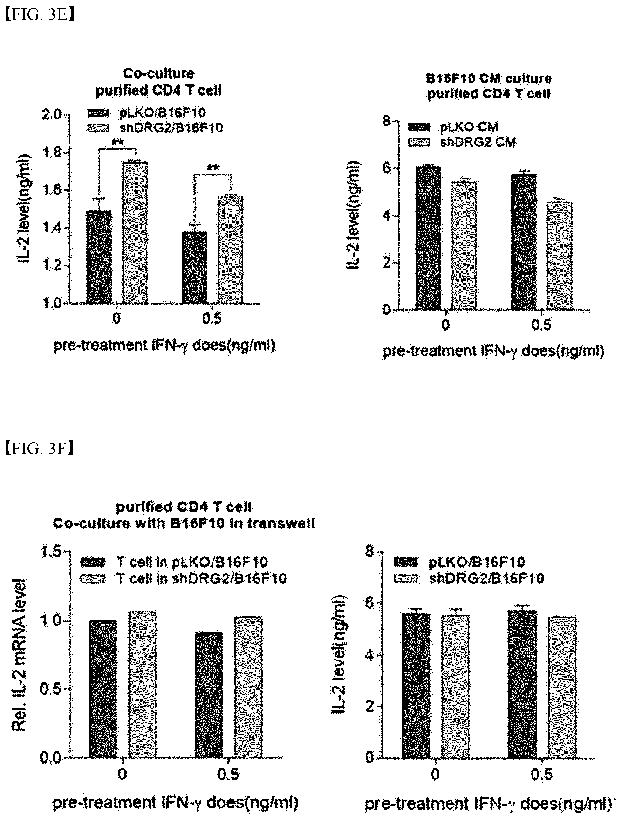

FIGS. 3E and 3F illustrate the result of confirming whether there is a difference in the activity of T cells *in vitro* as in the *in vivo* experiment results of Example 2. Cancer cells suppress the activity of T cells through direct contact using a surface protein such as PD-L1, but may also regulate T cells without direct contact by secreting cytokines. Therefore, the amounts of IL-2 were measured when cancer cells and T cells are co-cultured to maintain direct contact (left view of FIG 3E), when T cells were cultured using a cancer cell culture solution (conditioned media) from which cancer cells had been removed (right view of FIG 3E), and when T cells and cancer cells were co-cultured without direct contact using transwell plates (left view (RT-qPCR measurement results) and right view (ELISA measurement results) of FIG3F). As illustrated in FIGS. 3E and 3F, it was confirmed that the amount of IL-2 was increased only when T cells and cancer cells were in direct contact (left view of FIG 3E), and the amount of IL-2 did not show a significant difference when T cells and cancer cells were not in direct contact. Through this, it was confirmed that the activity of T cells was increased by direct contact.

Through the above results, it was confirmed that cancer cells with a decreased level of DRG2 increased the expression of PD-L1 by IFN-gamma, and T cells were activated only when there is direct contact with T cells.

### Example 4. Confirmation of effect of decrease in expression of DRG2 on PD-L1 function

In order to confirm why the expression level of PD-L1 is increased, but PD-L1 does not normally function in cancer with decreased levels of DRG2, the degree of binding between PD-1 which is a surface protein of T cells and PD-L1 which is a surface protein of cancer cells was first confirmed. The results are illustrated in FIGS. 4A to 4C.

As illustrated in FIG 4A, as a result of confirming the amount of cancer cells binding to the PD-1 protein using a flow cytometry analyzer when cancer cells were treated with the PD-1 protein, it was confirmed that the contact with PD-1 was further reduced in a sample of shDRG2/B16F10+IFN-gamma compared to pLKO/B16F10+IFN-gamma.

FIG 4B illustrates the results of re-confirming the amount of PD-L1 in the cells used in FIG 4A, confirming that when the level of DRG2 was decreased, the amount of PD-L1 was increased.

FIG 4C illustrates the results of calculating the amount of contact with PD-1 confirmed in FIG 4A compared to the amount of PD-L1 confirmed in FIG 4B, confirming that when the level of DRG2 was decreased, the amount of contact with PD-1 was remarkably decreased compared to the level of PD-L1.

Through the above results, it could be confirmed that the expression of PD-L1 was increased in cancer with decreased levels of DRG2, but the amount of binding between PD-L1 and PD-1 of T cells was conversely decreased. That is, it could be confirmed that in the case of cancer cells with decreased levels of DRG2, the direct contact with T cells was suppressed to increase the activity of T cells, and in the case of PD-L1 with increased expression, the function thereof was not normally maintained.

### Example 5. Confirmation of mechanism of deterioration in PD-L1 function in cancer cells according to decrease in DRG2 level

In order to confirm a mechanism by which a decrease in the level of DRG2 increases the expression of PD-L1, but suppresses the function of PD-L1, the degree of glycosylation of PD-L1 and the location of PD-L1 present in cancer cells were confirmed. The results are illustrated in FIGS. 5A to 5D.

FIG 5A illustrates the results (left) of confirming the degree of glycosylation of PD-L1 by western blot and the results (right) of quantifying the same, and it is known that the efficiency of interaction with PD-1 is reduced when the glycosylation of PD-L1 does not occur. As illustrated in FIG 5A, it was confirmed that in the case of complete glycosylation of PD-L1, PD-L1 with a size of 55 kDa was observed, but when an incompletely glycosylated part is cleaved by Endo H, a band was observed at a size of 30 to 35 kDa. However, it was confirmed that even when the level of DRG2 was decreased, there was still a large amount of fully glycosylated PD-L1 that was not cleaved by Endo H. Through the above results, it could be confirmed that when the level of DRG was decreased, the deterioration in function of PD-L1 was not exhibited due to the incompleteness of glycosylation.

FIG 5B illustrates the results of confirming the intracellular location of PD-L1. As illustrated in FIG 5B, it was confirmed that the proportion of PD-L1 present in cells was high in cancer cells with decreased levels of DRG2. Through the above results, it means that PD-L1 cannot migrate out of the cell by endocytosis and is present inside the cell, and for this reason, it could be confirmed that T cells could not be activated because the efficiency of interaction with PD-1 was reduced.

FIG 5C illustrates experimental results using SKOV3, which is a human ovarian cancer cell line. As illustrated in FIG 5C, it was confirmed that the expression of PD-L1 was increased by IFN-gamma even in human cancer cells with decreased levels of siDRG2 using siDRG2 (left bottom view), but PD-L1 was not normally located on the cell surface.

Through the above results, it could be confirmed that the expression of PD-L1 was increased in cancer with decreased levels of the DRG2 gene and/or DRG2 protein, but the binding ratio between PD-L1 and PD-1 on the surface of T cells is decreased because PD-L1 cannot be normally located on the cell surface, and PD-L1 did not normally function through this. Through this, it could be confirmed that a generally used agent for cancer immunotherapy, that is, an anti-PD-L1 antibody, an anti-PD-1 antibody, and the like did not normally work, and for this reason, only 20 to 30% of the PD-L1 positive cancer patients exhibited a therapeutic effect of the agent for cancer immunotherapy.

Therefore, it could be confirmed that it is possible to select a patient who may exhibit an effect of the treatment with an agent for cancer immunotherapy by measuring the level of a DRG2 gene or DRG2 protein in a biological sample, and in particular, an agent for cancer immunotherapy is generally administered to a PD-L1 positive cancer patient, but a patient for the administration of the agent for cancer immunotherapy can be more effectively selected by measuring the level of the DRG2 gene or DRG2 protein in the PD-L1 positive cancer patient. A composition for predicting a therapeutic response or prognosis for the agent for cancer immunotherapy of the present invention, or a method for providing information using the same is used to remarkably enhance the therapeutic efficiency of the agent for cancer immunotherapy, and thus is expected to effectively reduce the pain and treatment costs of a cancer patient.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

A composition for predicting a therapeutic response or prognosis for the agent for cancer immunotherapy of the present invention, or a method for providing information using the same can confirm a therapeutic response to the agent for cancer immunotherapy with high accuracy compared to the determination of whether to administer the agent for cancer immunotherapy using existing PD-L1. Therefore, the composition or the method can more accurately determine whether to administer the agent for cancer immunotherapy to various cancer patients, and can enhance a therapeutic effect through this, and thus is expected to effectively reduce the pain and treatment costs of the cancer patients.

## Claims

1. A composition for predicting a therapeutic response or prognosis for an agent for cancer immunotherapy, comprising a preparation that measures the level of a developmentally-regulated GTP-binding protein 2 (DRG2) gene or DRG2 protein.

2. The composition of claim 1, wherein the preparation that measures the level of the DRG2 gene is a primer or probe that specifically binds to the DRG2 gene.

3. The composition of claim 1, wherein the preparation that measures the level of the DRG2 protein is an antibody or aptamer that specifically binds to the DRG2 protein.

4. The composition of claim 1, wherein the agent for cancer immunotherapy is a drug that specifically binds to programmed cell death protein 1 (PD-1) or programmed death-ligand 1 (PD-L1).

5. The composition of claim 4, wherein the drug that specifically binds to PD-1 is an anti-PD-1 antibody.

6. The composition of claim 5, wherein the anti-PD-1 antibody is pembrolizumab, nivolumab or cemiplimab.

7. The composition of claim 4, wherein the drug that specifically binds to PD-L1 is an anti-PD-L1 antibody.

8. The composition of claim 7, wherein the anti-PD-L1 antibody is atezolizumab, avelumab or durvalumab.

9. The composition of claim 1, wherein the composition is for predicting a therapeutic response or prognosis for an agent for cancer immunotherapy against PD-L1 positive cancer.

10. The composition of claim 1, wherein the composition predicts cancer with decreased expression levels of the DRG2 gene or DRG2 protein compared to a control as cancer with a low therapeutic response to an agent for cancer immunotherapy or cancer with a poor prognosis.

11. The composition of claim 1, wherein the composition predicts PD-L1 positive cancer with decreased expression levels of the DRG2 gene or DRG2 protein compared to a control as cancer with a low therapeutic response to an agent for cancer immunotherapy or cancer with a poor prognosis.

12. The composition of claim 1, wherein decreased expression of the DRG2 gene or DRG2 protein shows a decrease in the level of PD-L1 expressed on the surface of cancer cells.

13. The composition of claim 12, wherein the decrease in the expression level of PD-L1 on the surface of cancer cells shows a decrease in binding between cancer cell PD-L1 and T cell PD-1.

14. The composition of claim 1, wherein decreased expression of the DRG2 gene or DRG2 protein shows an increased level of PD-L1 in cancer cells, and an increase in the number of CD8 T cells or activity thereof.

15. The composition of claim 1, wherein the level of the DRG2 gene or DRG2 protein is measured in a biological sample isolated from a cancer patient.

16. The composition of claim 15, wherein the biological sample is any one or more selected from the group consisting of cancer cells, cancer tissues, blood, plasma, serum, bone marrow, saliva, urine, and stool.

17. A kit for predicting a therapeutic response or prognosis for an agent for cancer immunotherapy, comprising the composition of any one of claims 1 to 16.

18. A composition for companion diagnosis for predicting a therapeutic response or prognosis for an agent for cancer immunotherapy in PD-L1 positive cancer, comprising a preparation that measures the level of a developmentally-regulated GTP-binding protein 2 (DRG2) gene or DRG2 protein.

19. A kit for companion diagnosis for an agent for cancer immunotherapy in PD-L1 positive cancer, comprising the composition for companion diagnosis for the agent for cancer immunotherapy in PD-L1 positive cancer of claim 18.

20. A method for providing information for predicting a therapeutic response or prognosis for an agent for cancer immunotherapy, the method comprising: measuring the level of a developmentally-regulated GTP-binding protein 2 (DRG2) gene or DRG2 protein in a biological sample isolated from a cancer patient.

21. The method of claim 20, wherein when the level of the DRG2 gene or DRG2 protein is decreased compared to a control, it is predicted that the therapeutic response to the agent for cancer immunotherapy is low or the prognosis is poor.

22. A method for providing information for predicting a therapeutic response or prognosis for an agent for cancer immunotherapy in PD-L1 positive cancer, the method comprising: measuring the level of a developmentally-regulated GTP-binding protein 2 (DRG2) gene or DRG2 protein in a biological sample isolated from a PD-L1 positive cancer patient.

23. The method of claim 22, wherein when the level of the DRG2 gene or DRG2 protein is decreased compared to a control, it is predicted that the therapeutic response to the agent for cancer immunotherapy is low or the prognosis is poor.

24. A method for treating cancer, the method comprising: a) measuring the level of a developmentally-regulated GTP-binding protein 2 (DRG2) gene or DRG2 protein in a biological sample isolated from a cancer patient; b) selecting a patient in which a level of the DRG2 gene or DRG2 protein is decreased compared to a control; and c) administering an agent for cancer immunotherapy to the selected patient.

25. A use of a composition comprising a preparation which measures the level of a developmentally-regulated GTP-binding protein 2 (DRG2) gene or DRG2 protein for predicting a therapeutic response or prognosis for an agent for cancer immunotherapy.
